# EUROPÄISCHE PATENTSCHRIFT

(11) **EP 1 601 293 B1**
(45) Veröffentlichungstag und Bekanntmachung des Hinweises auf die Patenterteilung: **24.02.2010**
(21) Anmeldenummer: 04716594.9
(22) Anmeldetag: 03.03.2004
(51) Int. Cl.: A61B 17/02

(54) **MEDIZINISCHES INSTRUMENTARIUM ZUM SCHAFFEN EINES OPERATIVEN ARBEITSRAUMES BEI KIEFEROPERATIONEN**
MEDICAL EQUIPMENT FOR CREATING A SURGICAL SPACE FOR ORAL SURGERY
APPAREIL MEDICAL PERMETTANT D'OBTENIR UN ESPACE DE TRAVAIL OPERATIONNEL LORS D'OPERATIONS DE LA MACHOIRE

(30) Priorität: 06.03.2003 DE 10310978
(43) Veröffentlichungstag der Anmeldung: 07.12.2005
(73) Patentinhaber: Karl Storz GmbH & Co. KG, 78532 Tuttlingen (DE)
(72) Erfinder: OBERLÄNDER, Martin, 78532 Tuttlingen (DE); REHBEIN, Stefan, 72505 Krauchenwies (DE); IRION, Klaus, M., 78576 Emmingen-Liptingen (DE); SAUER, Michael, 78532 Tuttlingen (DE); REINERT, Siegmar, 72074 Tübingen (DE); KRIMMEL, Michael, 72076 Tübingen (DE); UTZ, Eberhard, 72074 Tübingen (DE); HOFFMANN, Jürgen, 72076 Tübingen (DE)
(74) Vertreter: Heuckeroth, Volker
(86) Internationale Anmeldenummer: PCT/EP2004/002139
(87) Internationale Veröffentlichungsnummer: WO 2004/078046

(56) Entgegenhaltungen:
- DE-A- 2 503 404
- DE-A- 10 161 328

## Beschreibung

Die Erfindung betrifft ein medizinisches Instrumentarium zum Schaffen eines operativen Arbeitsraumes im menschlichen oder tierischen Körper auf minimal-invasivem Wege bei Kieferoperationen.

Operationen am Unterkiefer, insbesondere im Bereich des Kiefergelenks, die bspw. infolge von Frakturen des Unterkiefers erforderlich werden, müssen bisher häufig im Rahmen einer offenen Chirurgie durchgeführt werden. Bei solchen offenen Operationen werden vor dem Ohr des Patienten ausgedehnte Hautschnitte in der Wange ausgeführt, um den Unterkiefer an der Operationsstelle freizulegen und die erforderlichen Operationsmaßnahmen unter Sichtkontrolle mit dem bloßen Auge durchführen zu können.

Offene Operationen besitzen jedoch ein erhöhtes Komplikationspotential. Durch den relativ aufwendigen Zugang von außen können bspw. Verletzungen des Gesichtsnervs (Nervus Facialis) auftreten, die für den Patienten bleibende Lähmungen der Gesichtsmuskulatur zur Folge haben können. Neben diesen gesundheitlichen Risiken bestehen für den Patienten aufgrund der ausgedehnten Hautschnitte außerdem Beeinträchtigungen in ästhetischer Hinsicht durch entsprechende Narbenbildungen.

Um von den herkömmlichen offenen Operationsmethoden abzugehen und das Operationstrauma zu verringern, ist in der DE 197 17 977 A1 ein Instrumentarium beschrieben, mit dem Operationen am Unterkiefer bzw. Kiefergelenksoperationen im Wege der minimalinvasiven Chirurgie durchgeführt werden können. Mit diesem bekannten Instrumentarium kann in einem endoskopischen Verfahren eine Knochenplatte zur Osteosynthese am Unterkiefer angebracht werden, um eine Fraktur des Unterkiefers bzw. des Kiefergelenks zu versorgen.

Das bekannte Instrumentarium weist ein Endoskop sowie einen Endoskopzusatz auf, der das Endoskop sowie die Knochenplatte aufnimmt. Der Endoskopzusatz mit dem Endoskop wird über die Mundhöhle durch eine in der Mundhöhle geschaffene künstliche öffnung am Kiefer eingebracht. Über das Endoskop kann dann durch den Mund (transoral) beobachtet werden.

Das bekannte Instrumentarium umfaßt ferner ein Verbindungsrohr, das von außen durch die Wange über eine separate Inzision an die Operationsstelle eingeführt wird. Dieses Verbindungsrohr wird mit dem Endoskopzusatz im Bereich dessen distalen Endes fest verbunden.

Der Nachteil dieses bekannten Instrumentariums besteht darin, daß die Knochenplatte über die endoskopische Zusatzvorrichtung, die direkt mit dem Endoskop gekoppelt ist, an die Operationsstelle gebracht wird. Dadurch führen die notwendigen Bewegungen zur Ausrichtung und Fixierung der Knochenplatte auf dem zu fixierenden Kieferbereich zu einer unwillkürlichen und ständigen Änderung des Sichtfeldes, was die optische Kontrolle des Vorgangs erheblich erschwert. Des weiteren setzt die Befestigung des von außen durch die Wange eingesetzten Verbindungsrohrs mit der endoskopischen Zusatzvorrichtung voraus, daß die distale Seite des Verbindungsrohrs mit dem distalen Teil der endoskopischen Zusatzvorrichtung und der Knochenplatte zusammentrifft, was durch die zwei verschiedenen Zugänge, nämlich den transoralen Zugang einerseits und den Zugang durch die Wange, nicht oder nur zufällig gegeben ist.

Ein besonderer Nachteil des bekannten Instrumentariums besteht jedoch darin, daß mit dem bekannten Instrumentarium im Operationsgebiet kein präformierter Arbeitsraum geschaffen werden kann, der ein übersichtliches Arbeiten ermöglicht, was für ein sicheres Vorgehen bei der Operation erforderlich ist.

Aus dem Dokument DE 101 61 328 A ist eine Haltevorrichtung zum Abhalten der Wange oder Lippen bei oralchirurgischen oder kieferchirurgischen Operationen oder zahnärztlicher Behandlung bekannt. Die Haltevorrichtung weist einen Schaft auf, an dessen distalem Ende abgewinkelt ein Hakenteil angeordnet ist. An dem Hakenteil ist ein plattenförmiges flächiges Element als Wangenhalter angeordnet, der einen Aufnahmeschlitz für das Hakenteil aufweist und entlang des Hakenteils verschiebbar und durch Reibungskraft feststellbar ist.

Ferner ist aus dem Dokument DE 25 03 404 A eine Spreizvorrichtung für die Zahn- oder Mundhöhlenbehandlung bekannt, die das Aufspreizen der Wundränder, der Wangen des Patienten und der Knochenschleimhautlappen bei operativen Eingriffen zur Reinigung bzw. Säuberung in der Kieferchirurgie gestattet und darüber hinaus auch bei anderen Behandlungen sowohl des unteren als auch des oberen Kiefers anwendbar ist. Dieses bekannte Instrumentarium weist am proximalen Ende einen Handgriff auf, von dem sich distal ein Schaft erstreckt, dessen distales Ende umgebogen ist, und an dem ein plattenförmiges flächiges Element festlegbar ist. Das plattenförmige flächige Element dient zum Zurückhalten von Wundrändern und der Wange des Patienten im Bereich der Operationswunde.

Der Erfindung liegt daher die Aufgabe zugrunde, ein medizinisches Instrumentarium der eingangs genannten Art bereitzustellen, mit dem auf minimal-invasivem Wege bei Kieferoperationen ein operativer Arbeitsraum im menschlichen oder tierischen Körper geschaffen werden kann, so daß Kiefergelenksoperationen auf minimal-invasivem Wege möglichst atraumatisch und schnell, einfach und sicher und mit Sichtkontrolle durchgeführt werden können.

Erfindungsgemäß wird diese Aufgabe durch ein medizinisches Instrumentarium zum Schaffen eines operativen Arbeitsraumes im menschlichen oder tierischen Körper auf minimal-invasivem Wege bei Kieferoperationen gelöst, mit einem Schaft, der durch eine Inzision in der Wange einführbar ist, und mit einem plattenförmigen flächigen Element, das durch den Mund einführbar ist, wobei an einem distalen Ende des Schafts und an dem plattenförmigen Element ein Verbindungsmechanismus vorhanden ist, der so ausgebildet ist, daß das plattenförmige Element am Schaft festlegbar ist, wobei der Schaft als der Schaft eines Endoskops ausgebildet ist, oder wobei der Schaft als Einführhilfe zum Einführen eines Endoskops hohl ausgebildet ist.

Das erfindungsgemäße Instrumentarium ermöglicht es, auf minimal-invasivem Wege einen operativen Arbeitsraum im Bereich des Unterkiefers bei Kiefer- bzw. Kiefergelenksoperationen zu schaffen, indem der Schaft durch eine minimale Inzision bzw. einen Einstich in der Wange eingeführt und am distalen Ende des Schafts, das dem Kiefer gegenüberliegt, das plattenförmige flächige Element festgelegt wird, das zuvor durch den Mund an die Operationsstelle zwischen dem Unterkiefer und der Wange eingebracht wurde. Durch Zurückziehen des Schaftes aus der Inzision kann nun das Weichgewebe der Wange vom Kiefer zurückgehalten werden, wodurch für die durchzuführende Operation, die dann durch den Mund hindurch durchgeführt werden kann, genügend freier Arbeitsraum für die Werkzeuge, die bei der Operation verwendet werden, zur Verfügung steht. Die Schaffung dieses Arbeitsraumes erfolgt aufgrund der plattenförmigen flächigen Ausgestaltung des Elements auch auf atraumatische Weise, da sich das Element flächig an das Weichgewebe anlegt und Verletzungen des Gewebes dadurch ausgeschlossen sind. Das plattenförmige flächige Element stellt mit anderen Worten ein Retraktorelement dar, das über den Schaft von extrakorporal durch eine minimale Inzision bedient werden kann.

Der Schaft ist als Schaft eines Endoskops ausgebildet oder als Einführhilfe zum Einführen eines Endoskops hohl ausgebildet.

Hierbei ist von Vorteil, daß der Schaft nicht nur die Funktion der Schaffung des Arbeitsraumes erfüllt, sondern gleichzeitig auch die Funktion einer Sichtkontrolle im Wege einer endoskopischen Visualisierung des Operationsgebietes. Wenn der Schaft der Schaft eines Endoskops ist, ist das plattenförmige Element entsprechend am distalen Ende des Endoskops festlegbar.

Bei einer Ausgestaltung des Schafts als Einführhilfe für ein Endoskop wird der Vorteil erzielt, daß über den Schaft eine Visualisierung ermöglicht wird, die insbesondere das Festlegen des plattenförmigen Elements am distalen Ende des Schafts wesentlich vereinfacht.

Der weitere Vorteil dieser Maßnahme besteht darin, daß das plattenförmige Element nicht selbst am Endoskop festgelegt werden muß, so daß als Endoskop ein herkömmliches Endoskop verwendet werden kann, ohne daß bauliche Änderungen am Endoskop vorgenommen werden müssen, um das plattenförmige Element am Endoskop festzulegen.

Der noch weitere Vorteil dieser Ausgestaltung besteht darin, daß zwischen dem Schaft und dem Endoskop eine Relativbewegung ermöglicht wird, bspw. eine axiale und auch eine relative Drehbewegung zwischen dem Schaft und dem Endoskop, ohne daß das festgelegte plattenförmige Element dabei mitbewegt wird. Bspw. kann das distale Ende des Endoskops bei dieser Ausgestaltung auch über das distale Ende des Schafts und auch über das plattenförmige Element hinaus näher an die Operationsstelle herangeführt werden.

In einer bevorzugten Ausgestaltung ist das plattenförmige Element im wesentlichen schräg oder quer zum Schaft verlaufend am Schaft festlegbar.

Diese Maßnahme hat den Vorteil, daß damit der Arbeitsraum auf besonders effiziente Weise geschaffen werden kann, weil das Zurückziehen des Schafts zum Zurückziehen des plattenförmigen Elements im wesentlichen senkrecht zur Wange erfolgen kann, was dem Arzt die Handhabung des Instrumentariums wesentlich erleichtert.

In einer weiteren bevorzugten Ausgestaltung ist das plattenförmige Element von vorn mit dem äußeren distalen Ende des Schafts verbindbar.

Dies Maßnahme ist insbesondere dann von Vorteil, wenn durch den Schaft auch eine endoskopische Sichtkontrolle möglich ist, wie hiernach noch beschrieben wird, da das Festlegen des plattenförmigen Elements am Schaft dann unter Sichtkontrolle durch den Schaft hindurch erfolgen kann.

In einer weiteren bevorzugten Ausgestaltung ist die Form des plattenförmigen Elements an den Unterkiefer im Bereich des Kieferwinkels angepaßt, wobei sich das plattenförmige Element etwa von der Kieferbasis bis zum Kiefergelenk erstreckt.

Diese Maßnahme hat zum einen den Vorteil, daß das plattenförmige Element insgesamt großflächig ausgebildet ist, wodurch ein größerer Arbeitsraum im Bereich des Kieferwinkels geschaffen werden kann, weil mehr Gewebe vom Kieferknochen abgehalten werden kann, und zum anderen, daß im Fall, wie in einer noch zu beschreibenden Ausgestaltung vorgesehen ist, daß an dem plattenförmigen Element ein Saug- und/oder Spülsystem vorhanden ist, auch eine großräumige Spülung bzw. Absaugung am Operationsgebiet ermöglicht wird.

In einer weiteren bevorzugten Ausgestaltung befindet sich die Verbindungsstelle zwischen dem plattenförmigen Element und dem Schaft in einem Randbereich des plattenförmigen Elements, der im eingesetzten Zustand des plattenförmigen Elements dem Kiefergelenk benachbart ist.

Diese Maßnahme hat den Vorteil, daß die Inzision bzw. der Einstich zum Einführen des Schaftes in der Nähe des Kieferwinkels gesetzt werden kann, was zum einen das Risiko einer Verletzung des Gesichtsnervs noch weiter verringert. Darüber hinaus hat diese Ausgestaltung den Vorteil, daß der Schaft im Bereich des Ohres die durch den Mund durchgeführten Operationsschritte nicht behindert.

In einer weiteren bevorzugten Ausgestaltung ist am proximalen Ende des Schafts ein Haltegriff angeordnet, der seitlich von dem Schaft absteht.

Das Vorsehen eines Haltegriffs hat den Vorteil, daß die zum Zurückhalten des Gewebes zur Schaffung des Arbeitsraums erforderlichen Zugkräfte von der Bedienungsperson mit ausreichender Handkraft aufgebracht werden können. Der Haltegriff kann dabei vorteilhafterweise so mit dem Schaft verbunden sein, daß der Haltegriff im bestimmungsgemäßen Gebrauch zur Schläfe des Patienten hin zeigt.

Weiterhin ist es bevorzugt, wenn das plattenförmige Element mindestens im Verbindungsbereich mit dem Schaft transparent ist.

Diese Maßnahme hat den Vorteil, daß nicht nur der Vorgang des Festlegens des plattenförmigen Elements am Schaft durch das Endoskop beobachtet werden kann, sondern durch den transparenten Bereich kann mittels des Endoskops dann auch der Unterkiefer selbst beobachtet werden, insbesondere können die Operationsvorgänge, bspw. das Anbringen einer Knochenplatte am Kiefer, dann ebenfalls visualisiert werden.

In einer bevorzugten Alternative weist das plattenförmige Element zumindest im Verbindungsbereich mit dem Schaft eine Öffnung auf.

Auch in dieser Ausgestaltung wird der Vorteil erreicht, eine Visualisierung nicht nur des plattenförmigen Elements, sondern auch des dahinterliegenden Operationsgebietes zu ermöglichen, wobei diese Ausgestaltung den weiteren Vorteil besitzt, daß das distale Ende des Endoskops auch durch das plattenförmige Element hindurch näher an den Unterkiefer herangeführt werden kann, während das plattenförmige Element in seiner Funktion als Retraktorplatte zur Schaffung eines Arbeitsraumes vom Unterkiefer weiter beabstandet ist.

In einer weiteren bevorzugten Ausgestaltung ist an einem proximalen Ende des Schafts ein Anschluß zum Festlegen des Endoskops an dem Schaft angeordnet, wobei der Anschluß um die Längsachse des Schafts drehbar, vorzugsweise um 360° drehbar, ist.

Diese Maßnahme ist insbesondere dann von Vorteil, wenn das Endoskop eine Schräg- oder Seitblickoptik aufweist, so daß durch Drehen des Endoskops um seine Längsachse in alle Richtungen geblickt werden kann, ohne daß dabei der Schaft verdreht werden muß, so daß auch das plattenförmige Element dabei entsprechend seiner Funktion zur Schaffung eines Arbeitsraumes in Anlage mit dem wegzuhaltenden Weichgewebe nicht lageverändert werden muß.

In bevorzugten Ausgestaltungen des Verbindungsmechanismus zum Verbinden des plattenförmigen Elements mit dem Schaft weist der Verbindungsmechanismus einen Rastmechanismus und/oder einen Schraubmechanismus auf.

Diese Arten von Verbindungsmechanismen haben den Vorteil einer schnellen, einfachen Handhabung bei gleichzeitig sicherer Festlegung des plattenförmigen Elements am Schaft, wobei die Verbindungsmechanismen vorzugsweise allgemein so ausgeführt sind, daß sie sich von extrakorporal betätigen lassen, ohne daß umständliche Manipulationen im Operationsgebiet durchgeführt werden müssen.

In einer ersten bevorzugten konkreten Ausgestaltung des Verbindungsmechanismus weist der Schaft am distalen Ende einen sich im wesentlichen quer zur Längsrichtung des Schafts erstreckenden Zapfen auf, wobei das plattenförmige Element eine im wesentlichen komplementäre Öffnung aufweist, wobei der Zapfen in einer ersten Drehstellung in die Öffnung einsetzbar und durch Verdrehen in eine zweite Drehstellung gegen Herausziehen aus der Öffnung gesichert ist.

In dieser "Schlüsselloch"-Ausgestaltung läßt sich das plattenförmige Element mit dem Schaft intrakorporal durch eine einfach zu handhabende Steck- und Drehbewegung formschlüssig verrasten, wobei der Formschluß bei der erforderlichen Zugbelastung ein unerwünschtes Ablösen des plattenförmigen Elements von dem Schaft vermeidet.

Dabei ist es bevorzugt, wenn der Schaft einen Innenschaft, an dem der Zapfen ausgebildet ist, und einen Außenschaft aufweist, der zum Innenschaft axial verschiebbar, jedoch nicht verdrehbar ist, und der an einem distalen Ende zumindest einen gegenüber dem Zapfen um die Längsrichtung winkelig versetzten Fortsatz aufweist, der in der zweiten Drehstellung des Zapfens in die Öffnung eingreift.

Hierbei ist von Vorteil, daß der Außenschaft die formschlüssige Verbindung zwischen dem Zapfen des Innenschafts und der Öffnung des plattenförmigen Elements so sichert, daß der Schaft insgesamt drehfest mit dem plattenförmigen Element verbunden ist, und der Außenschaft vermeidet, daß bei Ausübung eines relativen Drehmoments zwischen dem Schaft und dem plattenförmigen Element der Zapfen unerwünscht in die erste Drehstellung zurückbewegt wird. Erst wenn der Außenschaft axial zurückgezogen wird, läßt sich der Innenschaft mit dem Zapfen von der zweiten Drehstellung wieder in die erste Drehstellung zurückbewegen, um das plattenförmige Element von dem Schaft abnehmen zu können.

Dabei ist weiterhin bevorzugt, wenn der Außenschaft mittels Federkraft nach distal vorgespannt ist.

Hierbei ist von Vorteil, daß der Außenschaft beim Verbinden des plattenförmigen Elements mit dem Schaft in der zweiten Drehstellung selbsttätig in die Öffnung einschnappt, wodurch die Handhabung des erfindungsgemäßen Instrumentariums beim Festlegen des plattenförmigen Elements an dem Schaft weiter vereinfacht wird.

In einer zweiten bevorzugten konstruktiven Ausgestaltung des Verbindungsmechanismus weist der Schaft einen Innenschaft auf, der an seinem distalen Ende eine Gabel aufweist, wobei das plattenförmige Element zwei Gewindesegmente aufweist, und wobei die Gabel mit den Gewindesegmenten in verschränkter Anordnung unverdrehbar zusammensteckbar ist, und wobei der Schaft weiterhin einen Außenschaft aufweist, dessen distales Ende mit den Gewindesegmenten verschraubbar ist.

Auch bei dieser Ausgestaltung ist der Schaft zweiteilig ausgebildet, wobei der Innenschaft gemäß einer Ausgestaltung eine Verdrehsicherung zwischen dem plattenförmigen Element und dem Schaft gewährleistet, während der Außenschaft das plattenförmige Element an dem Schaft in axialer Richtung festlegt.

Es versteht sich jedoch, daß der Schaft auch vorteilhafterweise für eine einfache Konstruktion einteilig ausgebildet und lediglich mit dem plattenförmigen Element verschraubbar sein kann.

In einer dritten bevorzugten konstruktiven Ausgestaltung weist der Schaft einen Innenschaft und einen Außenschaft auf, wobei der Außenschaft mit dem plattenförmigen Element verschraubbar ist, und wobei der Innenschaft am Außenschaft mittels eines Befestigungselements festlegbar und durch Lösen des Befestigungselements aus dem Außenschaft herausziehbar ist.

Im Unterschied zu den zuvor beschriebenen Ausgestaltungen wird hier nur der Außenschaft an dem plattenförmigen Element festgelegt, während der Innenschaft lediglich indirekt über das Befestigungselement und den Außenschaft lagefest zu dem plattenförmigen Element gehalten ist.

In einer weiteren bevorzugten Ausgestaltung weist das plattenförmige Element zumindest eine Saug- und/oder Spülleitung mit zumindest einer Saug- und/oder Spülöffnung auf.

In dieser Ausgestaltung hat das plattenförmige Element vorteilhafterweise zusätzlich die Funktion eines Saug- und/oder Spülinstruments, so daß auf die Einführung eines separaten Saug- und/oder Spülinstruments in das Operationsgebiet verzichtet werden kann, wodurch in dem Arbeitsraum möglichst wenig verschiedene Instrumente gleichzeitig vorhanden sind, die den Operationsvorgang behindern könnten. Durch Saugen und Spülen kann insbesondere die Sichtkontrolle durch das zuvor beschriebene Endoskop aufrechterhalten werden, indem mit klarer Spülflüssigkeit gespült und Gewebe- und Blutreste abgesaugt werden.

In einer weiteren bevorzugten Ausgestaltung weist der Schaft zumindest einen Saug- und/oder Spülkanal auf.

Hierbei ist von Vorteil, daß auch der Schaft eine weitere Funktion erhält, nämlich die Zuführung von Spülflüssigkeit und/oder die Absaugung von Flüssigkeiten, Blut und Gewebestücken aus dem Operationsgebiet.

Vorzugsweise wird durch den Schaft durch die Wange hindurch (transbukkal) gespült, während über das plattenförmige Element durch den Mund (transoral) abgesaugt wird.

In Verbindung mit einer der zuvor genannten Ausgestaltungen, wonach der Schaft einen Innenschaft und einen Außenschaft aufweist, wobei der Innenschaft am Außenschaft mittels eines Befestigungselements festlegbar ist, ist es in einer weiteren bevorzugten Ausgestaltung vorgesehen, wenn das Befestigungselement ein Saug- und/oder Spülanschluß zum Anschließen einer Saug- und/oder Spülleitung ist.

Hierbei ist von Vorteil, daß auf ein separates Befestitungselement zum Festlegen des Innenschafts am Außenschaft verzichtet werden kann, weil der Saug- und/oder Spülanschluß diese Funktion übernimmt, wodurch der konstruktive Aufwand des Schafts gering gehalten wird.

In einer weiteren bevorzugten Ausgestaltung weist das plattenförmige Element zumindest einen Positionssensor zum Erfassen der Position und/oder des Verbindungsbereichs des plattenförmigen Elements mit dem Schaft auf.

Der zumindest eine Positionssensor kann anstelle des oder zusätzlich zu einer visuellen Kontrolle dienen, um die Lage des plattenförmigen Elements im Operationsgebiet zu kontrollieren, und/oder um das Festlegen des plattenförmigen Elements am Schaft zu vereinfachen.

Dabei ist es bevorzugt, wenn der zumindest eine Positionssensor ein elektro-magnetischer Sensor ist.

Ein elektro-magnetischer Sensor hat den Vorteil, daß die Signalübertragung im Unterschied zu Licht auch ungehindert durch Gewebe hindurch erfolgen kann.

In einer weiteren bevorzugten Ausgestaltung weist das plattenförmige Element zumindest ein lichtabstrahlendes Element zum Abstrahlen von Licht durch die Wange hindurch auf.

Auch diese Ausgestaltung hat den Vorteil, daß über die Abstrahlung von Licht von dem plattenförmigen Element durch die Wange hindurch erfaßt werden kann.

Weitere Vorteile und Merkmale ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: eine schematische perspektivische Darstellung eines Instrumentariums zum Schaffen eines operativen Arbeitsraums bei Kieferoperationen, wobei das Instrumentarium im bestimmungsgemäßen Gebrauch vereinfacht dargestellt ist;
- Fig. 2: eine Seitenansicht eines Schafts eines Instrumentariums für die in Fig. 1 dargestellte Verwendung gemäß einem weiteren Ausführungsbeispiel;
- Fig. 3: eine Seitenansicht eines plattenförmigen Elements zur Verwendung mit dem Schaft in Fig. 2;
- Fig. 4: das plattenförmige Element in Fig. 3 in einer perspektivischen, gegenüber Fig. 3 um etwa 180° gedrehten Darstellung;
- Fig. 5: eine Explosionsdarstellung des gesamten Instrumentariums gemäß Figuren 2 bis 4 in perspektivischer Darstellung;
- Figuren 6a) - d): perspektivische Teilansichten des Verbindungsmechanismus zwischen dem plattenförmigen Element gemäß Figuren 3 und 4 und dem Schaft gemäß Fig. 2;
- Fig. 7: eine perspektivische, im Maßstab verkleinerte Darstellung des Instrumentariums gemäß Fig. 5 während des Festlegens des plattenförmigen Elements an dem Schaft;
- Fig. 8: eine Fig. 7 entsprechende Darstellung des Instrumentariums, wobei das plattenförmige Element nunmehr an dem Schaft festgelegt ist;
- Figuren 9a) und b): Darstellungen des Verbindungsmechanismus zum Verbinden des plattenförmigen Elements an dem Schaft, teilweise im Längsschnitt, wobei Fig. 9a) der Darstellung in Fig. 6b) und Fig. 9b) der Darstellung in Fig. 6d) entspricht;
- Fig. 10: eine perspektivische Explosionsdarstellung eines Instrumentariums gemäß einem weiteren Ausführungsbeispiel;
- Fig. 11: eine Seitenansicht des Schafts des Instrumentariums in Fig. 10 in Alleinstellung;
- Fig. 12: eine Detaildarstellung des Verbindungsmechanismus zum Verbinden des plattenförmigen Elements des Instrumentariums in Fig. 10 mit dem zugehörigen Schaft gemäß Fig. 10 bzw. Fig. 11 im vergrößerten Maßstab, wobei Fig. 12 den Verbindungsmechanismus vor dem Festlegen des plattenförmigen Elements an dem Schaft zeigt;
- Fig. 13: eine Fig. 12 entsprechende Darstellung, teilweise im Längsschnitt, wobei das plattenförmige Element nunmehr am Schaft festgelegt ist;
- Fig. 14: eine perspektivische Darstellung eines Instrumentariums gemäß einem weiteren Ausführungsbeispiel, wobei das plattenförmige Element des Instrumentariums vom Schaft getrennt dargestellt ist;
- Fig. 15: eine perspektivische Explosionsdarstellung des Schafts des Instrumentariums in Fig. 14 ohne das plattenförmige Element;
- Fig. 16: ein distales Ende des Schafts des Instrumentariums in Fig. 14 bzw. 15 im Längsschnitt und im vergrößerten Maßstab, wobei am distalen Ende des Schafts eine Kappe angebracht ist; und
- Fig. 17: eine perspektivische Darstellung der Kappe in Fig. 16 in Alleinstellung.

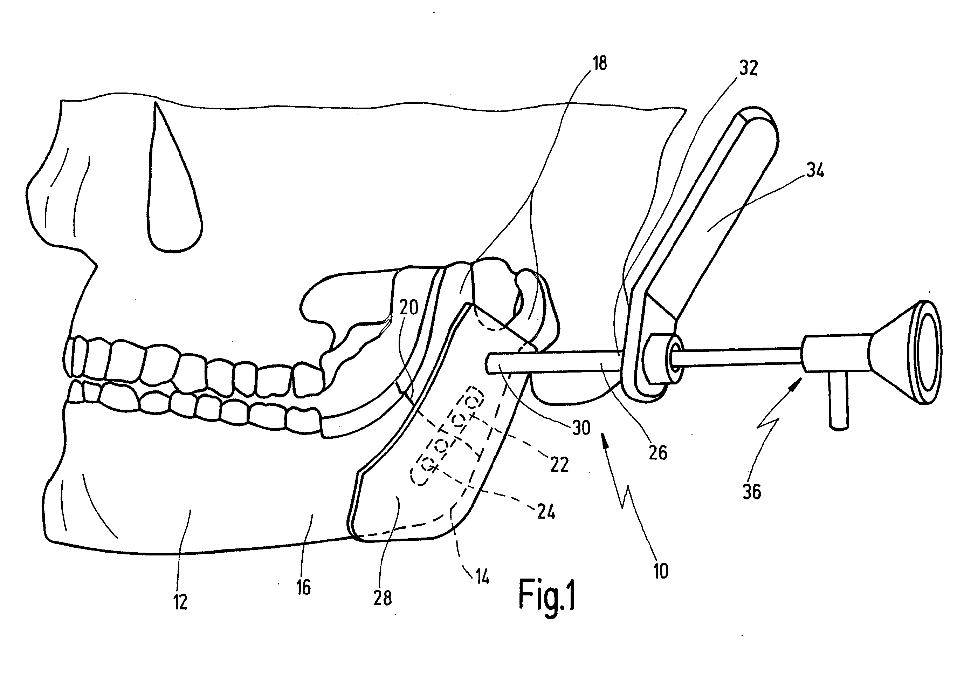

In Fig. 1 ist ein mit dem allgemeinen Bezugszeichen 10 versehenes medizinisches Instrumentarium zum Schaffen eines operativen Arbeitsraumes im menschlichen oder tierischen Körper auf minimal-invasivem Wege bei Kieferoperationen dargestellt. Das Instrumentarium 10 wird insbesondere bei Kiefergelenksoperationen verwendet.

In Fig. 1 ist ferner ein Unterkiefer 12 eines Patienten dargestellt, wobei der Kieferwinkel mit 14, die Kieferbasis mit 16 und das Kiefergelenk mit 18 bezeichnet ist. Eine Kiefergelenksoperation kann bspw. in Folge einer Fraktur 20 des Unterkiefers im Bereich zwischen dem Kieferwinkel 14 und dem Kiefergelenk 18 erforderlich werden. Im Rahmen dieser Operation wird die Fraktur 20 mittels einer Knochenplatte 22 versorgt, die die Fraktur 20 überspannend auf dem Unterkiefer 12 mittels Schrauben 24 aufgeschraubt wird.

Das Instrumentarium 10 weist allgemein einen Schaft 26 sowie ein plattenförmiges flächiges Element 28 auf. Das plattenförmige flächige Element 28 ist an einem distalen Ende 30 des Schafts 26 mittels eines später noch zu beschreibenden Verbindungsmechanismus festlegbar, und zwar nachdem das Element 28 transoral eingesetzt und der Schaft 26 transbukkal eingeführt worden ist, wobei das Festlegen des Elements 28 an dem Schaft 26 extrakorporal, d.h. von außen, betätigbar ist.

Das plattenförmige Element 28 ist dabei im wesentlichen schräg oder quer zum Schaft 26 verlaufend an diesem festgelegt, wie in Fig. 1 dargestellt ist.

Das plattenförmige Element 28 ist hinsichtlich seiner Form an den Unterkiefer 12 im Bereich des Kieferwinkels 14 angepaßt und erstreckt sich etwa von der Kieferbasis 16 bis zum Kiefergelenk 18. "An die Form des Unterkiefers 12" angepaßt bedeutet dabei, daß das plattenförmige Element 28 entsprechend der natürlichen Wölbung der Oberfläche des Unterkiefers 12 im Bereich von der Kieferbasis 16 bis zum Kiefergelenk 18 geringfügig gewölbt ist.

Wie aus Fig. 1 weiter hervorgeht, befindet sich die Verbindungsstelle zwischen dem plattenförmigen Element 28 und dem Schaft 26 in der Nähe des Randes des plattenförmigen Elementes 28, der dem Kieferwinkel 28 benachbart ist.

Bei einer Kiefergelenkoperation wird der Schaft 26 durch die Wange (transbukkal) durch eine minimale Inzision vor dem Ohr eingeführt. In der Mundhöhle wird in der dem Operationsbereich benachbarten Schleimhaut ein Schnitt angebracht. Das plattenförmige Element 28 wird dann transoral durch den Schnitt in der Schleimhaut zwischen den Unterkieferknochen 12 im Bereich des Kieferwinkels 14 und dem (Muskel-)Gewebe der Wange eingebracht. Das plattenförmige Element 28 wird anschließend von vorn mit der distalen Spitze des Schafts 26 verbunden, wie später noch beschrieben wird. Durch Zurückziehen des Schaftes 26 aus der Inzision in der Wange wird dann das plattenförmige Element 28 gegen das außen anliegende Gewebe zurückgezogen, wodurch ein Arbeitsraum zwischen der dem Unterkiefer 12 zugewandten Seite des plattenförmigen Elements 28 und dem Unterkiefer 12 geschaffen wird, in dem dann die Knochenplatte 22 mit entsprechenden Werkzeugen sicher und schnell angebracht werden kann.

Um das Zurückziehen des plattenförmigen Elements 28 mittels des Schafts 26 zu erleichtern, ist an einem proximalen Ende 32 des Schafts 26 ein Haltegriff 34 angeordnet, der seitlich von dem Schaft 26 absteht und etwa mindestens die Länge einer Handbreite besitzt. Der Haltegriff 34 zeigt im Einsatz-Zustand des Instrumentariums 10 etwa in Richtung der Schläfe des Patienten.

Der Schaft 26 des Instrumentariums 10 dient des weiteren zur Sichtkontrolle des Operationsgebietes. Während der Schaft 26 dazu selbst als Schaft eines Endoskops ausgebildet sein kann, indem bspw. in dem Schaft 26 eine Endoskopoptik vorhanden ist oder der Schaft 26 als Schaft eines Videoendoskops ausgebildet sein kann, ist der Schaft 26 in dem vorliegenden Ausführungsbeispiel hohl ausgebildet und dient somit als Einführhilfe zum Einführen eines Endoskops 36.

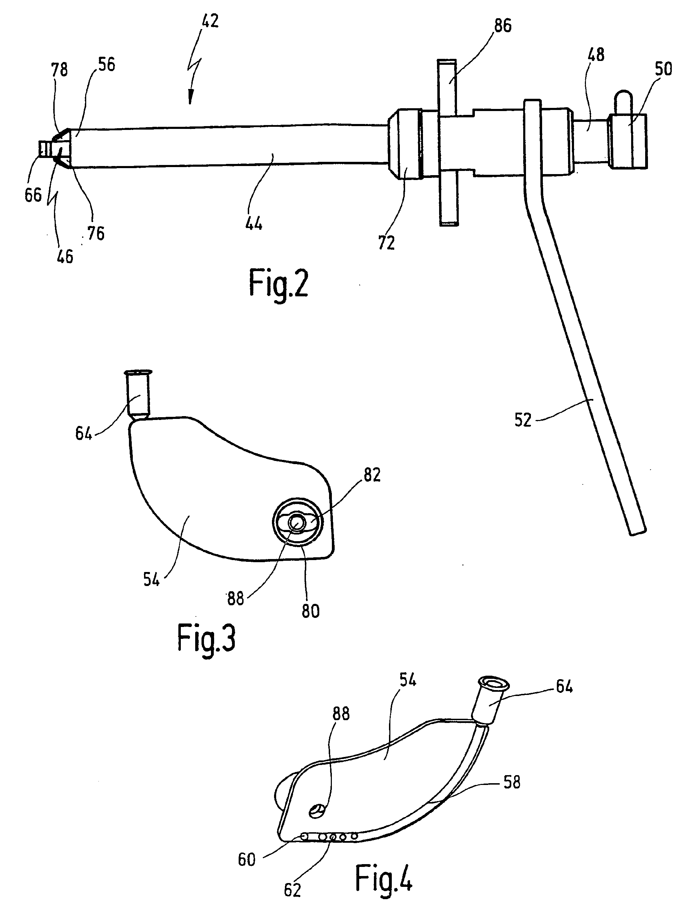

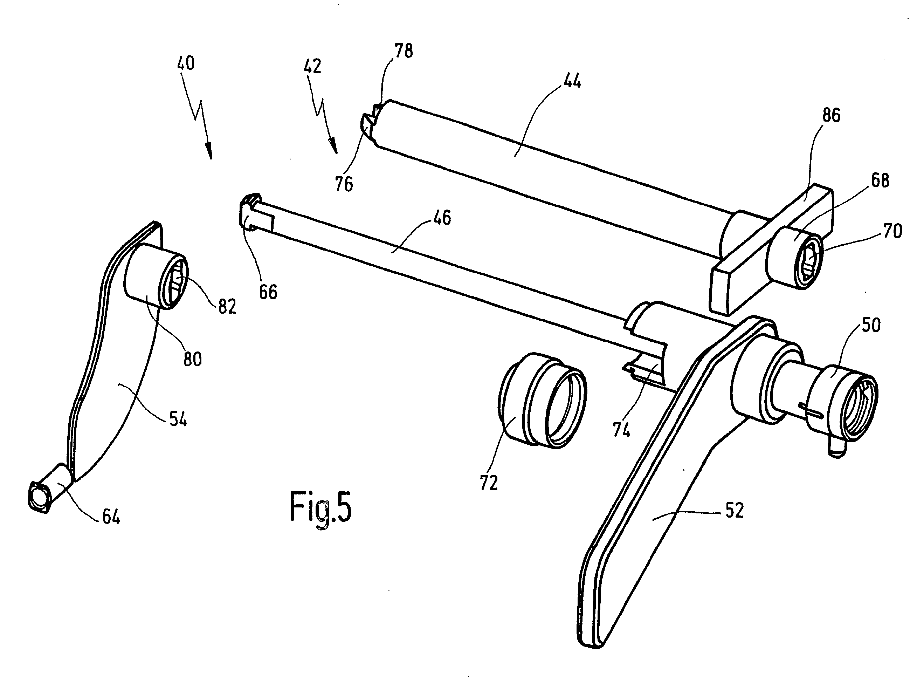

In Figuren 2 bis 9 ist gegenüber der Prinzipdarstellung des Instrumentariums 10 ein Ausführungsbeispiel eines Instrumentariums 40 (vgl. Fig. 5) in detaillierterer Weise dargestellt, das hiernach beschrieben wird, und das anstelle des Instrumentariums 10 in gleicher Weise wie in Fig. 1 dargestellt verwendet werden kann.

Das Instrumentarium 40 weist dem Schaft 26 des Instrumentariums 10 entsprechend einen Schaft 42 auf, der durch einen Außenschaft 44 und einen Innenschaft 46 gebildet wird. Dabei ist der Außenschaft 44 gemäß Fig. 5 von dem Innenschaft 46 abnehmbar. Der Schaft 42 dient wie der Schaft 26 als Einführhilfe bspw. für das Endoskop 36 in Fig. 1, und weist an einem proximalen Ende 48 einen Anschluß 50 auf, durch den das Endoskop 36 in den Schaft 42 einführbar ist, und mittels dem das Endoskop 36 an dem Schaft 42 festgelegt werden kann. Der Anschluß 50 ist um die Längsachse des Schafts 32 um 360° drehbar, so daß das Endoskop 36 zusammen mit dem Anschluß 50 um die Längsachse des Schafts 42 gedreht werden kann, ohne daß der Schaft 42 als Ganzes gedreht werden muß.

Der Anschluß 50 ist dabei an dem Innenschaft 46 angeordnet, d.h. der Innenschaft 46 nimmt das Endoskop 36 auf und ist entsprechend durchgehend hohl ausgebildet.

Am proximalen Ende 48 des Schafts 42 ist des weiteren ein Haltegriff 52 entsprechend dem Haltegriff 34 des Instrumentariums 10 in Fig. 1 angeordnet, der gemäß Fig. 5 am Innenschaft 46 befestigt ist.

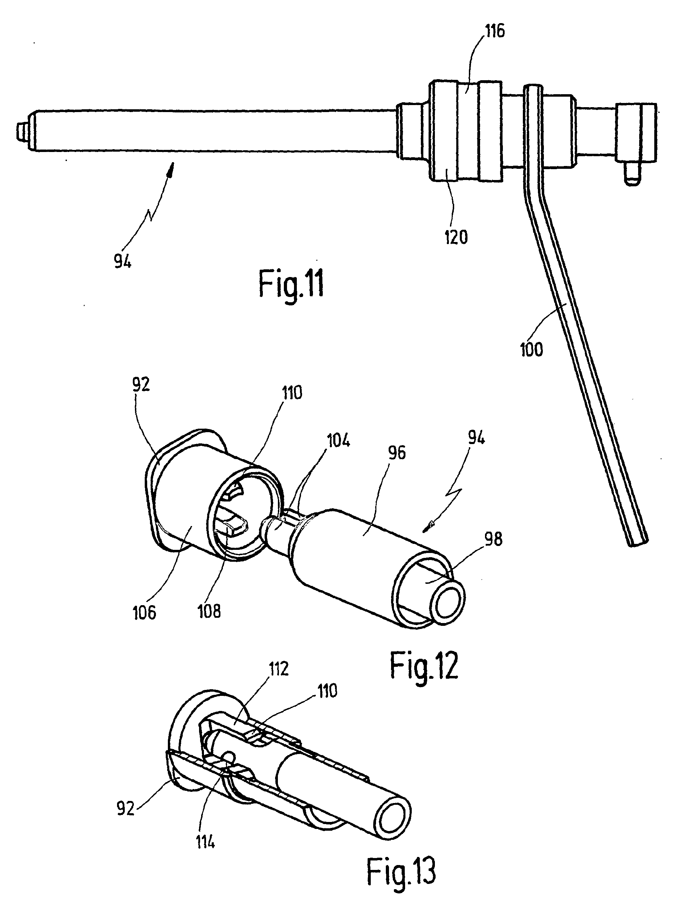

Das Instrumentarium 40 weist des weiteren ein dem plattenförmigen Element 28 des Instrumentariums 10 in Fig. 12 entsprechendes plattenförmiges flächiges Element 54 auf, das an einem distalen Ende 56 des Schafts 42 über einen hiernach noch zu beschreibenden Verbindungsmechanismus festlegbar ist.

Das plattenförmige Element 54 weist eine Saugleitung 58 auf, die auf einer Seite des plattenförmigen Elements 54 angeordnet ist, die beim bestimmungsgemäßen Gebrauch des Instrumentariums 40 gemäß Fig. 1 dem Unterkiefer 12 zugewandt ist. Die Saugleitung 58, die sich entlang eines unteren Randes des plattenförmigen Elements 54 erstreckt, weist an ihrem Ende eine Saugöffnung 60 sowie ggf. an ihrer Außenseite weitere Saugöffnungen 62 auf. An ihrem im bestimmungsgemäßen Gebrauch der Mundöffnung zugewandten Ende weist die Saugleitung 58 einen Anschluß 64 zum Anschließen eines Saugschlauches (nicht dargestellt) auf. Die Saugleitung 58 kann jedoch auch als Spülleitung verwendet werden, wenn dies gewünscht ist.

Ausgehend von Fig. 5 wird nachfolgend das Zusammensetzen des Schafts 42 und das Festlegen des plattenförmigen Elements 54 an dem Schaft 42 beschrieben.

Am distalen Ende 56 des Schafts 42, genauer gesagt am distalen Ende des Innenschafts 46, ist ein Zapfen 68 angeordnet, der sich quer zum Innenschaft 46 erstreckt und mit diesem etwa die Form eines T bildet. Der Außenschaft 44 weist an seinem proximalen Ende einen hülsenartigen Fortsatz 68 auf, der eine zu dem Zapfen 66 im wesentlichen komplementäre Öffnung 70 aufweist. Der Außenschaft 44 wird vor Beginn der Operation vom distalen Ende des Innenschafts 46 her auf diesen aufgeschoben, wobei sich der Außenschaft 44 aufgrund der Formgebung der Öffnung 70 nur in einer bestimmten Drehstellung relativ zu dem Innenschaft 46 über den Zapfen 66 auf den Innenschaft 46 aufschieben läßt. Anschließend wird eine Überwurfmutter 72 auf den Außenschaft 44 aufgeschoben und gemäß Fig. 2 mit dem Innenschaft 46 verschraubt. Der Außenschaft 44 ist dann drehfest mit dem Innenschaft 46 verbunden, kann jedoch weiterhin gegen den Innenschaft 46 axial verschoben werden. Der Außenschaft 44 ist mittels Federkraft, bspw. durch eine in einem hülsenartigen Fortsatz 74 des Innenschaftes 46 angeordnete nicht dargestellte Feder, in seine in Fig. 2 dargestellte maximal nach distal verschobene Lage vorgespannt, und kann gegen die Federkraft nach proximal zurückgezogen werden.

Der Außenschaft 44 weist an seinem distalen Ende zwei um 180° zueinander versetzt angeordnete Fortsätze 76, 78 auf, die im auf den Innenschaft 46 montierten Zustand des Außenschaftes 44 zu den Zapfen 66 des Innenschafts 46 winklig versetzt sind, und zwar um 90°.

Nach der zuvor beschriebenen Vormontage des Schafts 42 kann nun der Schaft 42, wie in Verbindung mit Fig. 1 beschrieben, durch eine kleine Inzision vor dem Ohr durch die Wange eingeführt werden.

Das plattenförmige Element 54, das transoral über die Mundhöhle und den in Verbindung mit Fig. 1 beschriebenen Schnitt in der Schleimhaut an Ort und Stelle gebracht wird, weist einen hülsenartigen Fortsatz 80 auf, der eine Öffnung 82 aufweist, die im wesentlichen komplementär zu dem Zapfen 66 ausgebildet ist.

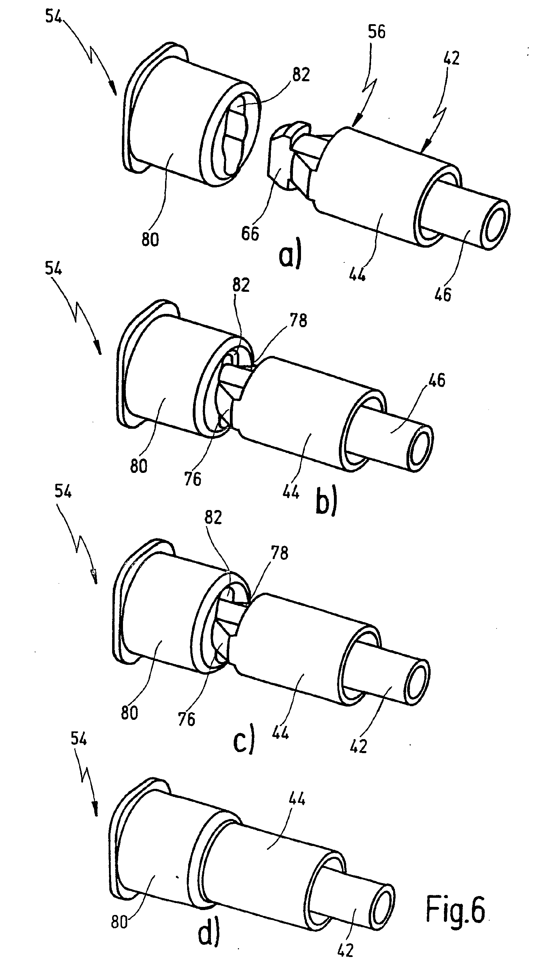

Mit Bezug auf Figuren 6 bis 9 wird nunmehr das Festlegen des plattenförmigen Elements 54 an den Schaft 42 beschrieben.

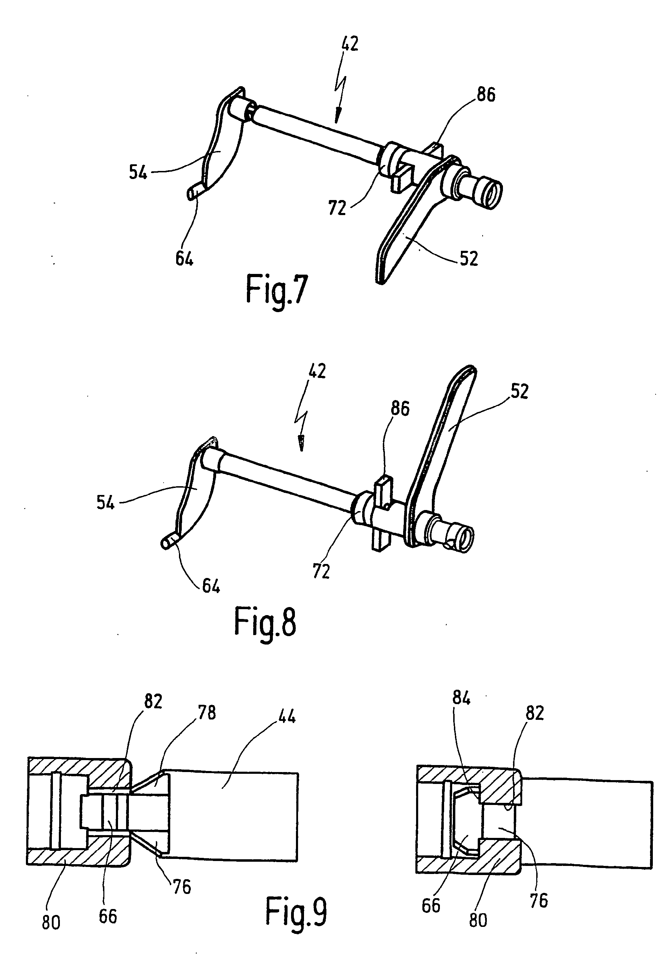

In Figuren 6a) bis 6d) ist das plattenförmige Element 54 lediglich im Bereich des Fortsatzes 80 und der Schaft 42 lediglich im Bereich seines distalen Endes 56 dargestellt, das Gleiche gilt für Figuren 9a) und 9b).

Ausgehend von Figur 6a) wird der Schaft 42 mit dem Zapfen 66 voran in die Öffnung 82 des Fortsatzes 80 in der Art eines Schlüssels eingeführt, was aufgrund der Formgebung des Zapfens 66 und der dazu im wesentlichen komplementären Ausbildung der Öffnung 82 nur in einer bestimmten Drehstellung des Schafts 42 relativ zu dem plattenförmigen Element 54 möglich ist. Dieser Zustand ist in Fig. 6b) bzw. Fig. 9a) dargestellt. Die Fortsätze 76 und 78 des Außenschafts 44 kommen dabei mit dem Rand der Öffnung 82 in Anlage, wie in Fig. 6b) bzw. Fig. 9a) dargestellt ist.

Durch weiteres Vorschieben des Schaftes 42 gemäß Fig. 6c) tritt der Zapfen 66 vollständig in die Öffnung 82 ein, während der Außenschaft 44 dabei relativ zu dem Innenschaft 46 zurückgeschoben wird. Diese Position ist außer in Fig. 6c) auch in Fig. 7 dargestellt. Ausgehend von dieser Stellung wird nun der Schaft 42 mittels des Haltegriffs 52 in eine zweite Drehstellung um die Längsachse des Schafts 42 gedreht (vgl. Fig. 8), so daß der Zapfen 66 an einer Hinterschneidung 84 der Öffnung 82 anliegend formschlüssig gegen ein Herausziehen gesichert ist. Der drehfest mit dem Innenschaft 46 verbundene Außenschaft 44 wird dabei ebenfalls um 90° verdreht, so daß die Fortsätze 76 und 78 dann in die Öffnung 82 einschnappen, wodurch der Schaft 42 insgesamt drehfest und zugfest mit dem plattenförmigen Element 54 verbunden ist. Dieser Zustand ist in Fig. 6d), Fig. 8 und Fig. 9b) dargestellt.

Zum Lösen des plattenförmigen Elements 54 von dem Schaft 42 nach Beendigung der Operation wird umgekehrt vorgegangen, wobei zunächst der Außenschaft 44 relativ zu dem Innenschaft 46 gegen die Federkraft zurückgezogen wird, wozu an dem Außenschaft 44 ein Griffelement 86 am proximalen Ende angeordnet ist. Nach dem Zurückziehen des Außenschafts 44 kann dann der gesamte Schaft 42 mittels des Haltegriffs 52 um 90° verdreht werden, wonach der Schaft 42 von dem plattenförmigen Element 54 abgezogen werden kann.

Bereits vor dem Festlegen des plattenförmigen Elements 54 an dem Schaft 42 kann in den Schaft 42 das Endoskop 36 in Fig. 1 eingeführt werden, so daß der Festlegevorgang durch das Endoskop 36 kontrolliert werden kann. In dem plattenförmigen Element 54 ist ferner mit der Öffnung 82 fluchtend eine Öffnung 88 vorhanden, durch die hindurch nach dem Festlegen des plattenförmigen Elements 54 an dem Schaft 42 mittels des Endoskops 36 die einzelnen Operationsschritte optisch kontrolliert bzw. visualisiert werden können. Das Endoskop 36 kann dabei, wenn dies sinnvoll ist, auch mit seinem distalen Ende durch die Öffnung 88 hindurchgeführt werden, so daß das Endoskop in unterschiedlichen Entfernungen zu dem Unterkiefer 12 eingestellt werden kann.

Alternativ zu der Öffnung 88, oder zusätzlich zu der Öffnung 88, kann das plattenförmige Element 54 auch zumindest im Bereich der Verbindung mit dem Schaft 44 transparent ausgeführt sein.

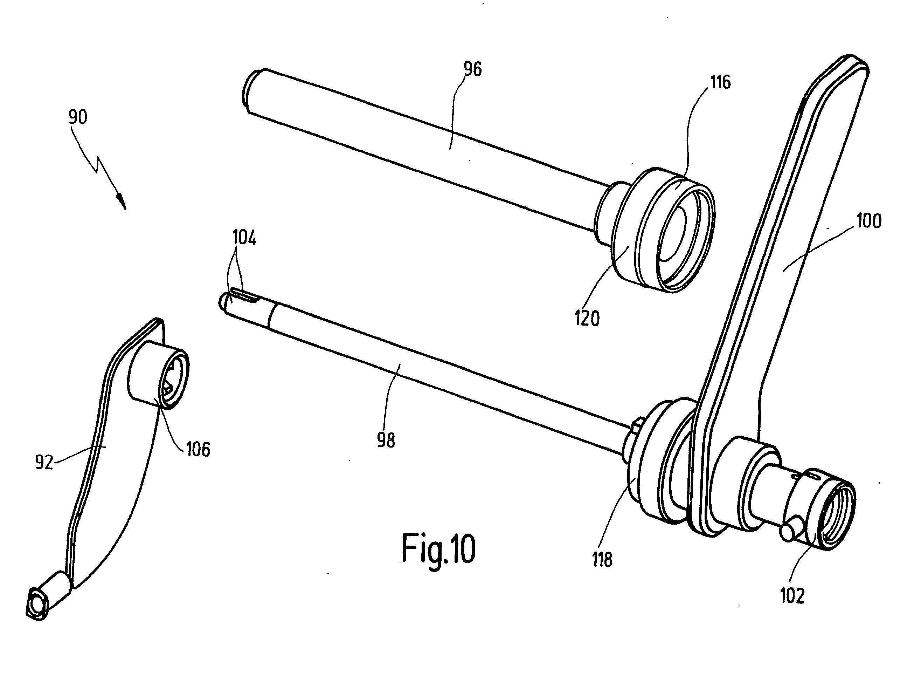

Während der zuvor in Verbindung mit Figuren 2 bis 9 beschriebene Verbindungsmechanismus ein Rastmechanismus ist, bei dem der Schaft 42 mit dem plattenförmigen Element 54 verrastbar ist, wird mit Bezug auf Figuren 10 bis 13 ein weiteres Ausführungsbeispiel eines Instrumentariums 90 beschrieben, bei dem der Verbindungsmechanismus zum Festlegen eines plattenförmigen Elements 92 an einem Schaft 94 im wesentlichen auf einem Schraubmechanismus beruht.

Soweit nachfolgend mit Bezug auf das Instrumentarium 90 nichts Gegenteiliges beschrieben wird, entspricht die Ausgestaltung des Instrumentariums 90 ansonsten dem Instrumentarium 10 bzw. dem Instrumentarium 30.

Der Schaft 94 weist wie bei dem vorherigen Ausführungsbeispiel einen Außenschaft 96 und einen Innenschaft 98 auf, sowie einen Haltegriff 100, der mit dem Innenschaft 98 verbunden ist, und einen Anschluß 102 zum Einführen und Festlegen bspw. des Endoskops 36 in Fig. 1, wobei der Anschluß 102 ebenfalls am Innenschaft 98 angeordnet ist.

Der Innenschaft 98 weist an seinem distalen Ende eine Gabel 104 auf, d.h. der Innenschaft 98 ist an seinem distalen Ende geschlitzt ausgebildet. Das plattenförmige Element 92 weist einen hülsenartigen Fortsatz 106 auf, in dessen Inneren zwei Gewindesegmente 108 und 110 um 180° zueinander versetzt angeordnet sind. Die Gewindesegmente 108 und 110 weisen auf ihrer Außenseite 112 ein Gewinde auf, wie für das Gewindesegment 110 in Fig. 13 dargestellt ist.

Der Außenschaft 96 weist an seinem distalen Ende ein Innengewinde 114 auf.

Ausgehend von Fig. 10 wird zunächst der Außenschaft 96 auf den Innenschaft 98 aufgeschoben, so daß der Schaft 94, wie in Fig. 11 dargestellt, zusammengesetzt vorliegt. Der Außenschaft 96 wird dabei mittels einer Überwurfmutter 116, die auf ein Gewindestück 118 am Innenschaft 98 aufschraubbar ist, am Innenschaft 98 festgelegt. Dabei bleibt jedoch der Außenschaft 96 relativ zu dem Innenschaft 98 frei drehbar, indem sich an die Überwurfmutter 116 ein Drehgelenk 120 anschließt. Um den so vormontierten Schaft 94 mit dem plattenförmigen Element 92 zu verbinden, wird der Schaft 94 mit der Gabel 104 voran in den Fortsatz 106 des plattenförmigen Elements 92 eingesteckt, wobei die Gabel in um 90° verschränkter Anordnung mit den Gewindesegmenten 108 und 110 zusammengesteckt wird, wie in Fig. 13 dargestellt ist. Der Innenschaft 96 ist sodann drehfest mit dem plattenförmigen Element 92 verbunden. Anschließend wird gemäß Fig. 13 der Außenschaft 96 mit seinem Innengewinde 114 auf die Gewindesegmente 108 und 110 aufgeschraubt, so daß anschließend der Schaft 94 auch in axialer Richtung an dem plattenförmigen Element 92 festgelegt ist. Das Lösen des Schafts 94 von dem plattenförmigen Element 94 geschieht entsprechend in umgekehrter Reihenfolge.

Der Haltegriff 100 ist in bezug auf die Gabel 104 so orientiert, daß im am Schaft 94 festgelegten Zustand des plattenförmigen Elements 92 der Haltegriff 100 im bestimmungsgemäßen Gebrauch des Instrumentariums 90 gemäß Fig. 1 zur Schläfe des Patienten zeigt.

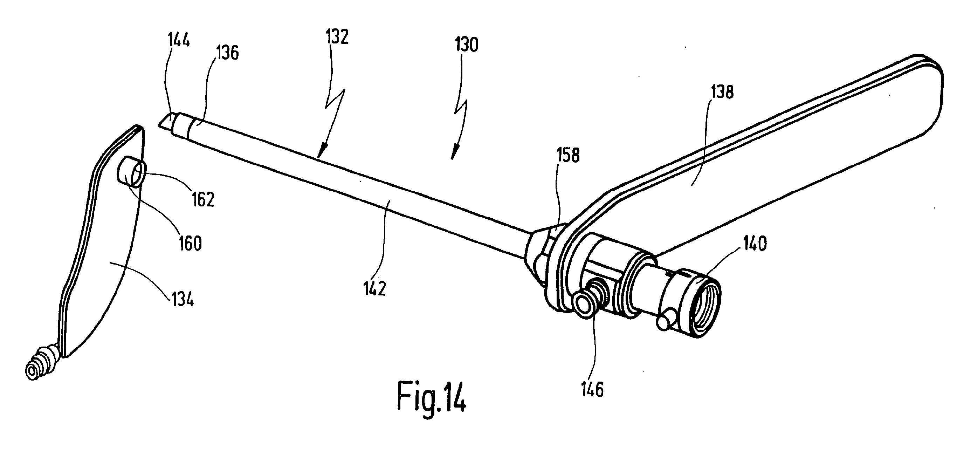

Mit Bezug auf Figuren 14 bis 17 wird ein noch weiteres Ausführungsbeispiel eines Instrumentariums 130 beschrieben, das entsprechend dem Instrumentarium 10 gemäß Fig. 1 verwendet werden kann.

Sofern nachfolgend nichts Gegenteiliges beschrieben wird, sind hiernach nicht beschriebene Merkmale des Instrumentariums 130 mit den Merkmalen der Instrumentarien 10, 40 und 90 identisch oder entsprechen diesen zumindest.

Das Instrumentarium 130 weist einen Schaft 132 sowie ein plattenförmiges Element 134 auf, das an einem distalen Ende 136 des Schafts 132 mittels eines hiernach noch zu beschreibenden Verbindungsmechanismus festlegbar ist.

Der Schaft 132 weist an seinem proximalen Ende einen seitlich von dem Schaft 132 abstehenden Haltegriff 138 auf. Am äußersten proximalen Ende weist der Schaft 132 einen Anschluß 140 zum Festlegen bspw. des Endoskops 36 in Fig. 1 am Schaft 132 auf, wobei das Endoskop 36 in den Schaft 132 einführbar ist, wie mit Bezug auf die vorherigen Ausführungsbeispiele bereits beschrieben wurde.

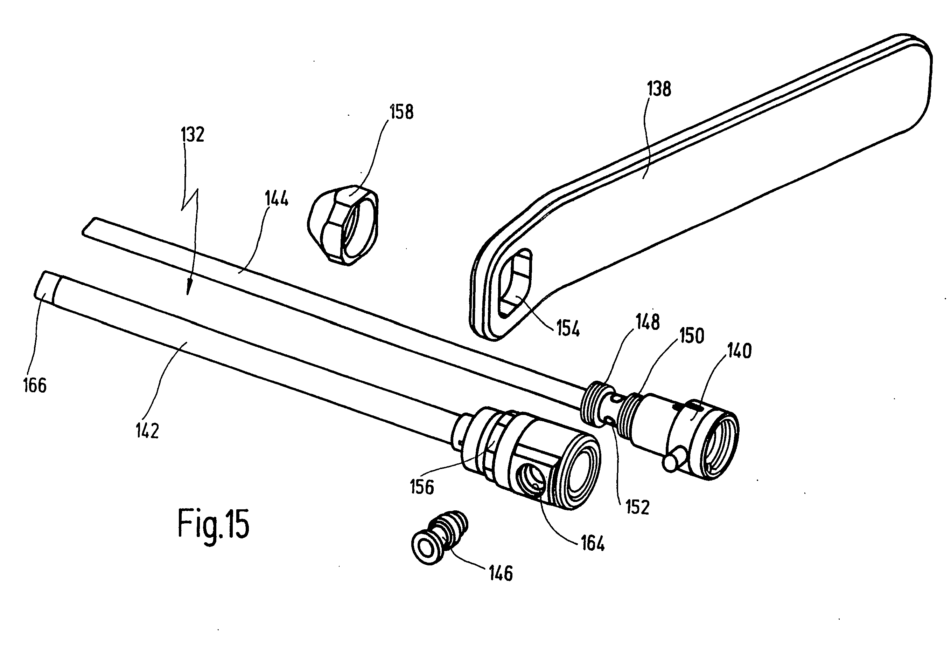

Der Schaft 132 weist einen Außenschaft 142 und einen Innenschaft 144 auf. Der Außenschaft 142 ist auf den Innenschaft 144 aufschiebbar, wie in Fig. 14 dargestellt ist. In Fig. 15 sind der Außenschaft 142 und der Innenschaft 144 im voneinander abgenommenen Zustand dargestellt.

Der Innenschaft 144, der an seinem proximalen Ende den Anschluß 140 aufweist, dient nicht nur als Einführhilfe für ein Endoskop, bspw. das Endoskop 36 in Fig. 1, sondern ist gleichzeitig als Spülschaft ausgebildet und weist einen nicht näher dargestellten entsprechenden Spülkanal auf.

Am proximalen Ende des Schafts 132 ist entsprechend ein Spülanschluß 146 angeordnet, der neben der Funktion eines Anschlusses für eine nicht dargestellte Spülleitung eine weitere Funktion hat, nämlich den Innenschaft 144 am Außenschaft 142 festzulegen, wie hiernach noch beschrieben wird. Dichtungen 148 und 150 dichten den Innenschaft 144 gegen den Außenschaft 142 ab. Zwischen den Dichtungen 148 und 150 sind im Innenschaft 144 Öffnungen 152 vorhanden, in eine von denen der Anschluß 146 einschraubbar ist, wobei die entsprechende Öffnung 152 dann als Eintrittsöffnung von Spülflüssigkeit in den Innenschaft 144 dient.

Der Haltegriff 138 ist vom Schaft 132 abnehmbar. Der Haltegriff 138 wird mittels einer Öffnung 152 auf den Außenschaft 142 aufgeschoben. Die Öffnung 154 ist im wesentlichen rechteckig ausgebildet, wobei an dem Außenschaft 142 ein dazu komplementärer Vierkant 156 vorhanden ist, auf die die Öffnung 154 formschlüssig in vier verschiedenen Drehstellungen aufgesetzt werden kann. Zur Befestigung des Haltegriffs 138 an dem Außenschaft 142 ist eine Überwurfmutter 158 vorgesehen.

Das plattenförmige Element 134 weist einen hülsenartigen Fortsatz 160 auf, der eine im wesentlichen zylindrische Öffnung 162 aufweist.

Vor der Operation wird der Schaft 132 durch Einschieben des Innenschafts 144 in den Außenschaft 142 zusammengesetzt. Anschließend wird der Anschluß 146 durch eine Öffnung 164 im proximalen Ende des Außenschafts 142 sowie in eine der Öffnungen 152 eingeschraubt, wodurch der Innenschaft 144 an dem Außenschaft 142 sowohl drehfest als auch axial unverschiebbar festgelegt ist. Der Haltegriff 138 wird auf den Außenschaft 142 aufgeschoben und mittels der Überwurfmutter 158 festgelegt.

Der so vormontierte Schaft 132 kann dann, wie mit Bezug auf Fig. 1 beschrieben, durch die Inzision vor dem Ohr in das Operationsgebiet eingeführt werden.

Der Außenschaft 142 weist an seinem distalen Ende ein Außengewinde 166 auf, während die Öffnung 162 des Fortsatzes 160 des plattenförmigen Elements 134 ein entsprechendes Innengewinde aufweist.

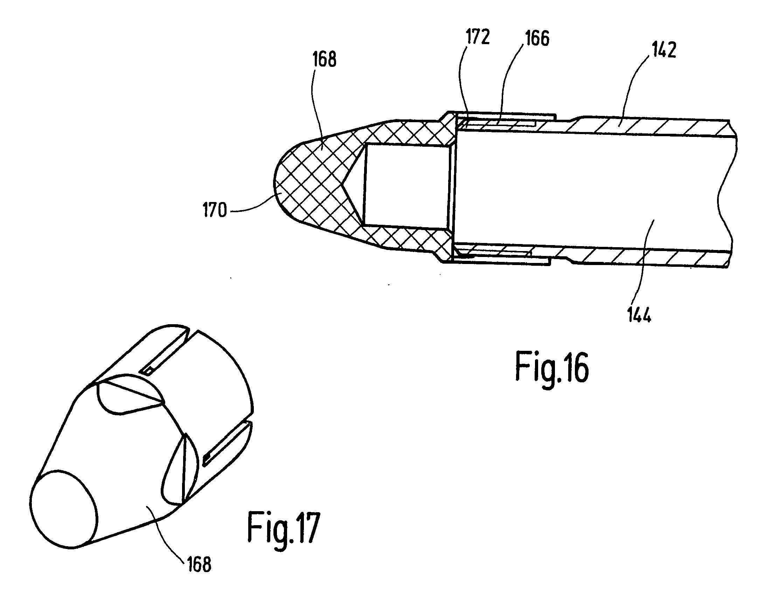

Vor dem Einführen des Schaftes 132 in die Inzision wird auf das Außengewinde 166 des Außenschaftes 142 eine Kappe 168 gemäß Fig. 17 wie in Fig. 16 dargestellt aufgeschraubt, um das Einführen des Schafts 132 in die Inzision zu erleichtern. Die Kappe 168 ist mit einem abgerundeten Ende 170 versehen, und deckt das Außengewinde 166 und die Spitze 172 des Schafts 132 ab, so daß die Kappe 168 das Einführen des Schafts 132 in die Inzision atraumatisch gestaltet.

Die Kappe 168 kann dann intrakorporal von der Mundhöhle her abgeschraubt werden, wonach der Schaft 132, genauer gesagt der Außenschaft 142, dann in das Gewinde der Öffnung 162 des Fortsatzes 160 des zuvor transoral eingeführten plattenförmigen Elements 134 eingeschraubt werden kann, wonach das plattenförmige Element 134 an dem Schaft 132 festgelegt ist.

Wie zuvor bereits beschrieben, kann der Haltegriff 138 durch entsprechendes Lösen der Überwurfmutter 158 in eine gewünschte Position gebracht werden, vorzugsweise so, daß der Haltegriff 138, wie in Fig. 1 mit Bezug auf das Instrumentarium 10 dargestellt ist, im bestimmungsgemäßen Gebrauch des Instrumentariums 130 zur Schläfe des Patienten hin gerichtet ist.

Es versteht sich, daß die vorstehend genannten Ausführungsbeispiele hinsichtlich ihrer konstruktiven Ausgestaltung auch miteinander kombiniert werden können. In weiteren nicht dargestellten Ausgestaltungen kann des weiteren an dem jeweiligen plattenförmigen Element 28, 54, 92 oder 134 zumindest ein Positionssensor zum Erfassen der Position und/oder des Verbindungsbereichs des jeweiligen plattenförmigen Elements mit dem jeweiligen Schaft 26, 42, 94 oder 132 vorhanden sein. Ein solcher Positionssensor ist vorzugsweise ein elektro-magnetischer Sensor.

Des weiteren ist es möglich, an den zuvor genannten plattenförmigen Elementen zumindest ein lichtabstrahlendes Element zum Abstrahlen von Licht durch die Wange des Patienten hindurch vorzusehen.

## Patentansprüche

1. Medizinisches Instrumentarium zum Schaffen eines operativen Arbeitsraumes im menschlichen oder tierischen Körper auf minimal-invasivem Wege bei Kieferoperationen, mit einem Schaft (26; 42; 94; 132), der durch eine Inzision in der Wange einführbar ist, und mit einem plattenförmigen flächigen Element (28; 54; 92; 134), das durch den Mund einführbar ist, wobei an einem distalen Ende des Schafts (26; 42; 94; 132) und an dem plattenförmigen Element (28; 54; 92; 134) ein Verbindungsmechanisnms vorhanden ist, der so ausgebildet ist, daß das plattenförmige Element (28; 54; 92; 134) am Schaft (26; 42; 94; 132) festlegbar ist, dadurch charakterisiert, dass der Schaft (26; 42; 94; 132) als der Schaft eines Endoskops ausgebildet ist, oder dass der Schaft (26; 42; 94; 132) als Einführhilfe zum Einführen eines Endoskops (36) hohl ausgebildet ist.

2. Instrumentarium nach Anspruch 1, wobei das plattenförmige Element (28; 54; 92; 134) in sich im wesentlichen schräg oder quer zum Schaft (26; 42; 94; 132) verlaufend am Schaft (26; 42; 94; 132) festlegbar ist.

3. Instrumentarium nach Anspruch 1 oder 2, wobei das plattenförmigen Element (28; 54; 92; 134) von vorn mit dem äußeren distalen Ende des Schafts (26; 42; 94; 132) verbindbar ist.

4. Instrumentarium nach einem der Ansprüche 1 bis 3, wobei die Form des plattenförmigen Elements (28; 54; 92; 134) an den Unterkiefer (12) im Bereich des Kieferwinkels (14) angepaßt ist und sich etwa von der Kieferbasis (16) bis zum Kiefergelenk (18) erstreckt.

5. Instrumentarium nach einem der Ansprüche 1 bis 4, wobei sich die Verbindungsstelle zwischen dem plattenförmigen Element (28; 54; 92; 134) und dem Schaft (26; 42; 94; 132) in einem Randbereich des plattenförmigen Elements (28; 54; 92; 134) befindet, der im eingesetzten Zustand des plattenförmigen Elemente (28; 54; 92; 134) dem Kiefergelenk (18) benachbart ist.

6. Instrumentarium nach einem der Ansprüche 1 bis 5, wobei am proximalen Ende des Schafts (26; 42; 94; 132) ein Haltegriff (34; 52; 100; 138) angeordnet ist, der seitlich von dem Schaft (26; 42; 94; 132) absteht.

7. Instrumentarium nach einem der Ansprüche 1 bis 6, wobei das plattenförmige Element (28; 54; 92; 134) zumindest im Verbindungsbereich mit dem Schaft transparent ist.

8. Instrumentarium nach einem der Ansprüche 1 bis 7, wobei das plattenförmige Element (28; 54; 92; 134) zumindest im Verbindungsbereich mit dem Schaft eine Öffnung (88) aufweist.

9. Instrumentarium nach einem der Ansprüche 1 bis 8, wobei an einem proximalen Ende des Schafts (26; 42; 94; 132) ein Anschluß (50; 102; 140) zum Festlegen des Endoskops (36) an dem Schaft angeordnet ist, wobei der Anschluß (50; 102; 140) um die Längsachse des Schafts (26; 42; 94; 132) drehbar, vorzugsweise um 360° drehbar ist.

10. Instrumentarium nach einem der Ansprüche 1 bis 9, wobei der Verbindungsmechanismus zum Verbinden des plattenförmigen Elements (28; 54) mit dem Schaft (26; 42) einen Rastmechanismus aufweist.

11. Instrumentarium nach einem der Ansprüche 1 bis 10, wobei der Verbindungsmechanismus zum Verbinden des plattenförmigen Elements (28; 92; 134) mit dem Schaft (26; 94; 132) einen Schraubmechanismus aufweist.

12. Instrumentarium nach Anspruch 10 oder 11, wobei der Schaft (42) am distalen Ende einen sich im wesentlichen quer zur Längsrichtung des Schafts (42) erstreckenden Zapfen (66) aufweist, wobei das plattenförmige Element (54) eine im wesentlichen komplementäre Öffnung (82) aufweist, wobei der Zapfen (66) in einer ersten Drehstellung in die Öffnung (82) einsetzbar und durch Verdrehen in eine zweite Drehstellung gegen Herausziehen aus der Öffnung (82) gesichert ist.

13. Instrumentarium nach Anspruch 12, wobei der Schaft (42) einen Innenschaft (46), an dem der Zapfen (66) ausgebindet ist, und einen Außenschaft (44) aufweist, der zum Innenschaft (46) axial verschiebbar, jedoch nicht verdrehbar ist, und der an einem distalen Ende zumindest einen gegenüber dem Zapfen (66) um die Längsrichtung winkelig versetzten Fortsatz (76, 78) aufweist, der in der zweiten Drehstellung des zapfens (66) in die Öffnung (82) eingreift.

14. Instrumentarium nach Anspruch 13, wobei der Außenschaft (44) mittels Federkraft nach distal vorgespannt ist.

15. Instrumentarium nach Anspruch 10 oder 11, wobei der Schaft (94) einen Innenschaft (98) aufweist, der an seinem distalen Ende eine Gabel (104) aufweist, und wobei das plattenfärmige Element (92) zumindest zwei Gewindesegmente (108, 110) aufweist, wobei die Gabel (104) mit den Gewindesegmenten (108, 110) in verschränkter Anordnung unverdrehbar zusammensteckbar ist, und wobei der Schaft (94) weiterhin einen Außenschaft (96) aufweist, dessen distales Ende mit den Gewindesegmenten (108, 110) verschraubbar ist.

16. Instrumentarium nach Anspruch 10 oder 11, wobei der Schaft (132) einen Innenschaft (144) und einen Außenschaft (142) aufweist, wobei der Außenschaft (142) mit dem plattenförmigen Element (134) verschraubbar ist, und wobei der Innenschaft (144) am Außenschaft (142) mittels eines Befestigungselements festlegbar und durch Lösen des Befestigungselements aus dem Außenschaft (142) herausziehbar ist.

17. Instrumentarium nach einem der Ansprüche 1 bis 16, wobei das plattenförmige Element (28; 54; 92; 134) zumindest eine Saug- und/oder Spülleitung (58) mit zumindest einer Saug- und/oder Spülöffnung (60, 62) aufweist.

18. Instrumentarium nach einem der Ansprüche 1 bis 17, wobei der Schaft (132) zumindest einen Saug- und/oder Spülkanal ausweist.

19. Instrumentarium nach Anspruch 16 und nach Anspruch 17 oder 18, wobei das Befestigungselement ein Saug- und/oder Spülanschluß (146) zum Anschließen einer Saug- und/oder Spülleitung ist.

20. Instrumentarium nach einem der Ansprüche 1 bis 19, wobei das plattenförmige Element (28; 54; 92; 134) zumindest einen Positionssensor zum Erfassen der Position und/oder des Verbindungsbereichs des plattenformigen Elements (28; 54; 92; 134) mit dem Schaft (26; 42; 94; 132) aufweist.

21. Instrumentarium nach Anspruch 20, wobei der zumindest eine Positionssensor ein elektro-magnetischer Sensor ist.

22. Instrumentarium nach einem der Ansprüche 1 bis 21, wobei das plattenförmigen Element (28; 54; 92; 134) zumindest ein lichtabstrahlendes Element zum Abstrahlen von Licht durch die wange hindurch aufweist.

## Claims

1. A medical instrument set for creating a surgical operating space in the human or animal body by minimally invasive way in operations on the jaw, comprising a shaft (26; 42; 94; 132) which can be introduced through an incision in the cheek, and a plate-shaped flat element (28; 54; 92; 134) which can be introduced through the mouth, a connection mechanism being provided at a distal end of the shaft (26; 42; 94; 132) and on the plate-shaped element (28; 54; 92; 134) and being designed such that the plate-shaped element (28; 54; 92; 134) can be secured on the shaft (26; 42; 94; 132), **characterized in that** the shaft (26; 42; 94; 132) is designed as the shaft of an endoscope, or that the shaft (26; 42; 94; 132) is designed hollow as an insertion aid for insertion of an endoscope (36).

2. The instrument set of claim 1, wherein the plate-shaped element (28; 54; 92; 134) can be secured on the shaft (26; 42; 94; 132) in such a way that it extends substantially obliquely or transversely with respect to the shaft (26; 42; 94; 132).

3. The instrument set of claim 1 or 2, wherein the plate-shaped element (28; 54; 92; 134) can be connected to the outer distal end of the shaft (26; 42; 94; 132) from the front.

4. The instrument set of anyone of claims 1 through 3, wherein the shape of the plate-shaped element (28; 54; 92; 134) is adapted to the lower jaw (12) in the area of the jaw angle (14) and extends approximately from the jaw base (16) to the jaw joint (18).

5. The instrument set of anyone of claims 1 through 4, wherein the connection point between the plate-shaped element (28; 54; 92; 134) and the shaft (26; 42; 94; 132) is located in an edge area of the plate-shaped element (28; 54; 92; 134), which edge area is adjacent to the jaw joint (18) when the plate-shaped element (28; 54; 92; 134) is in the inserted state.

6. The instrument set of anyone of claims 1 through 5, wherein the proximal end of the shaft (26; 42; 94; 132) is provided with a handle (34; 52; 100; 138) which protrudes laterally from the shaft (26; 42; 94; 132).

7. The instrument set of anyone of claims 1 through 6, wherein the plate-shaped element (28; 54; 92; 134) is transparent at least in the area of connection to the shaft.

8. The instrument set of anyone of claims 1 through 7, wherein the plate-shaped element (28; 54; 92; 134) has an opening (88) at least in the area of connection to the shaft.

9. The instrument set of anyone of claims 1 through 8, wherein a proximal end of the shaft (26; 42; 94; 132) is provided with an attachment piece (50; 102; 140) for securing the endoscope (36) on the shaft, the attachment piece (50; 102; 140) being able to rotate about the longitudinal axis of the shaft (26; 42; 94; 132), preferably through 360°.

10. The instrument set of anyone of claims 1 through 9, wherein the connection mechanism for connecting the plate-shaped element (28; 54) to the shaft (26; 42) has a catch mechanism.

11. The instrument set of anyone of claims 1 through 10, wherein the connection mechanism for connecting the plate-shaped element (28; 92; 134) to the shaft (26; 94; 132) has a screw mechanism.

12. The instrument set of claim 10 or 11, wherein the shaft (42) has, at the distal end, a plug (66) which extends substantially transversely with respect to the longitudinal direction of the shaft (42), the plate-shaped element (54) having a substantially complementary opening (82), and the plug (66) is able to be fitted into the opening (82) in a first position of rotation and, by rotating it to a second position of rotation, is secured against withdrawal from the opening (82).

13. The instrument set of claim 12, wherein the shaft (42) has an inner shaft (46), on which the plug (66) is formed, and an outer shaft (44) which is axially displaceable but not rotatable with respect to the inner shaft (46), and which has, at a distal end, at least one projection (76, 78) which is offset by an angle with respect to the plug (66) about the longitudinal direction and which, with the plug (66) in the second position of rotation, engages in the opening (82).

14. The instrument set of claim 13, wherein the outer shaft (44) is pretensioned in the distal direction by means of a spring force.

15. The instrument set of claim 10 or 11, wherein the shaft (94) has an inner shaft (98) which has a fork (104) at its distal end, and wherein the plate-shaped element (92) has at least two thread segments (108, 110), and the fork (104) can be coupled to the thread segments (108, 110) in a cross-over arrangement secure against rotation, and the shaft (94) moreover has an outer shaft (96) whose distal end can be screwed onto the thread segments (108, 110).

16. The instrument set of claim 10 or 11, wherein the shaft (132) has an inner shaft (144) and an outer shaft (142), the outer shaft (142) being able to be screwed onto the plate-shaped element (134), and the inner shaft (144) being able to be secured on the outer shaft (142) by means of a securing element and being able to be withdrawn from the outer shaft (142) by loosening of the securing element.

17. The instrument set of anyone of claims 1 through 16, wherein the plate-shaped element (28; 54; 92; 134) has at least one suction and/or irrigation line (58) with at least one suction and/or irrigation opening (60, 62).

18. The instrument set of anyone of claims 1 through 17, wherein the shaft (132) has at least one suction and/or irrigation channel.

19. The instrument set of claim 16 and of claim 17 or 18, wherein the securing element is a suction and/or irrigation attachment piece (146) for attachment of a suction and/or irrigation line.

20. The instrument set of anyone of claims 1 through 19, wherein the plate-shaped element (28; 54; 92; 134) has at least one position sensor for detecting the position and/or the area of connection of the plate-shaped element (28; 54; 92; 134) to the shaft (26; 42; 94; 132).

21. The instrument set of claim 20, wherein the at least one position sensor is an electromagnetic sensor.

22. The instrument set of anyone of claims 1 through 21, wherein the plate-shaped element (28; 54; 92; 134) has at least one light-emitting element for radiating light through the cheek.

## Revendications

1. Instrument médical pour créer un espace de travail opératoire dans le corps humain ou animal par des voies invasives minimales lors d'opérations de la mâchoire, comprenant une tige (26 ; 42 ; 94 ; 132) qui peut être introduite par une incision dans la joue, et un élément plat en forme de plaque (28 ; 54 ; 92 ; 134) qui peut être introduit à travers la bouche, sachant que sur une extrémité distale de la tige (26 ; 42 ; 94 ; 132) et sur l'élément en forme de plaque (28 ; 54 ; 92 ; 134), un mécanisme de liaison est présent, qui est réalisé de telle manière que l'élément en forme de plaque (28 ; 54 ; 92 ; 134) peut être fixé sur la tige (26 ; 42 ; 94 ; 132),
**caractérisé en ce que**
la tige (26 ; 42 ; 94 ; 132) est réalisée comme la tige d'un endoscope ou que la tige (26 ; 42 ; 94 ; 132) est réalisée de manière creuse pour aider à l'introduction d'un endoscope (36).

2. Instrument selon la revendication 1, sachant que l'élément en forme de plaque (28 ; 54 ; 92 ; 134) peut être fixé en soi sur la tige (26 ; 42 ; 94 ; 132) en étant essentiellement de biais ou de manière transversale par rapport à la tige (26 ; 42 ; 94 ; 132).

3. Instrument selon la revendication 1 ou 2, sachant que l'élément en forme de plaque (28 ; 54 ; 92 ; 134) peut être relié par l'avant à l'extrémité distale extérieure de la tige (26 ; 42 ; 94 ; 132).

4. Instrument selon l'une quelconque des revendications 1 à 3, sachant que la forme de l'élément en forme de plaque (28 ; 54 ; 92 ; 134) est adaptée à la mandibule (12) au niveau de l'angle de la mandibule (14) et s'étend environ de la base maxillaire (16) jusqu'à l'articulation maxillaire (18).

5. Instrument selon l'une quelconque des revendications 1 à 4, sachant que le point de liaison entre l'élément en forme de plaque (28 ; 54 ; 92 ; 134) et la tige (26 ; 42 ; 94 ; 132) se trouve dans une zone de bordure de l'élément en forme de plaque (28 ; 54 ; 92 ; 134) qui est adjacente à l'articulation maxillaire (18) quand l'élément en forme de plaque (28 ; 54 ; 92 ; 134) est installé.

6. Instrument selon l'une quelconque des revendications 1 à 5, sachant qu'une poignée de maintien (34 ; 52 ; 100 ; 138) est disposée sur l'extrémité proximale de la tige (26 ; 42 ; 94 ; 132), laquelle poignée fait saillie latéralement de la tige (26 ; 42 ; 94 ; 132).

7. Instrument selon l'une quelconque des revendications 1 à 6, sachant que l'élément en forme de plaque (28 ; 54 ; 92 ; 134) est transparent au moins dans la zone de liaison avec la tige.

8. Instrument selon l'une quelconque des revendications 1 à 7, sachant que l'élément en forme de plaque (28 ; 54 ; 92 ; 134) présente une ouverture (88) au moins dans la zone de liaison avec la tige.

9. Instrument selon l'une quelconque des revendications 1 à 8, sachant qu'un raccord (50 ; 102 ; 140) pour fixer l'endoscope (36) sur la tige est positionné sur une extrémité proximale de la tige (26 ; 42 ; 94 ; 132), sachant que le raccord (50 ; 102 ; 140) peut être tourné sur l'axe longitudinal de la tige (26 ; 42 ; 94 ; 132), de préférence de 360°.

10. Instrument selon l'une quelconque des revendications 1 à 9, sachant que le mécanisme de liaison pour relier l'élément en forme de plaque (28 ; 54) à la tige (26 ; 42) présente un mécanisme d'encliquetage.

11. Instrument selon l'une quelconque des revendications 1 à 10, sachant que le mécanisme de liaison pour relier l'élément en forme de plaque (28 ; 54 ; 92 ; 134) à la tige (26 ; 42 ; 94 ; 132) présente un mécanisme de vissage.

12. Instrument selon la revendication 10 ou 11, sachant que la tige (42) présente sur l'extrémité distale un tenon (66) s'étendant essentiellement transversalement à la direction longitudinale de la tige (42), l'élément en forme de plaque (54) présente une ouverture (82) essentiellement complémentaire, sachant que le tenon (66) peut être inséré dans une première position de rotation dans l'ouverture (82) et est sécurisé par rotation dans une deuxième position de rotation pour éviter qu'il ne sorte de l'ouverture (82).

13. Instrument selon la revendication 12, sachant que la tige (42) présente une tige interne (46) sur laquelle le tenon (66) est formé, et une tige externe (44) qui peut être poussée axialement en direction de la tige interne (46) mais ne peut pas être tournée et qui présente sur l'extrémité distale au moins un prolongement (76, 78) décalé angulairement par rapport au tenon (66), qui se met en prise dans l'ouverture (82) dans la deuxième position de rotation du tenon (66).

14. Instrument selon la revendication 13, sachant que la tige externe (44) est précontrainte vers la direction distale au moyen d'une force de ressort.

15. Instrument selon la revendication 10 ou 11, sachant que la tige (94) présente une tige interne (98) qui présente une fourche (104) sur son extrémité distale et sachant que l'élément en forme de plaque (92) présente au moins deux segments filetés (108, 110), sachant que la fourche (104) peut être insérée avec les segments filetés (108, 110) sans pouvoir tourner dans un agencement délimité, et sachant que la tige (94) présente en outre une tige externe (96) dont l'extrémité distale peut être vissée avec les segments filetés (108, 110).

16. Instrument selon la revendication 10 ou 11, sachant que la tige (132) présente une tige interne (144) et une tige externe (142), sachant que la tige externe (142) peut être vissée à l'élément en forme de plaque (134) et sachant que la tige interne (144) peut être fixée sur la tige externe (142) au moyen d'une élément de fixation et peut être tirée de la tige externe (142) par la séparation de l'élément de fixation.

17. Instrument selon l'une quelconque des revendications 1 à 16, sachant que l'élément en forme de plaque (28 ; 54 ; 92 ; 134) présente au moins une conduite d'aspiration et/ou de rinçage (58) avec au moins une ouverture d'aspiration et/ou de rinçage (60, 62).

18. Instrument selon l'une quelconque des revendications 1 à 17, sachant que la tige (132) présente au moins un canal d'aspiration et/ou de rinçage.

19. Instrument selon les revendications 16 et 17 ou 18, sachant que l'élément de fixation est un raccord d'aspiration et/ou de rinçage (146) pour raccorder une conduite d'aspiration et/ou de rinçage.

20. Instrument selon l'une quelconque des revendications 1 à 19, sachant que l'élément en forme de plaque (28 ; 54 ; 92 ; 134) présente au moins un capteur de position pour détecter la position et/ou la zone de liaison de l'élément en forme de plaque (28 ; 54 ; 92 ; 134) avec la tige (26 ; 42 ; 94 ; 132).

21. Instrument selon la revendication 20, sachant que l'au moins un capteur de position est un capteur électromagnétique.

22. Instrument selon l'une quelconque des revendications 1 à 22, sachant que l'élément en forme de plaque (28 ; 54 ; 92 ; 134) présente au moins un élément émettant de la lumière pour émettre de la lumière à travers la joue.
